# EUROPEAN PATENT SPECIFICATION

(11) **EP 4 275 708 B1**
(45) Date of publication and mention of the grant of the patent: **04.12.2024**
(21) Application number: 22172575.7
(22) Date of filing: 10.05.2022
(51) Int. Cl.: A61L 2/20, B67B 3/00

(54) **COMPACT STERILIZATION MACHINE FOR THE STERILIZATION OF CAPS**
KOMPAKTE STERILISATIONSMASCHINE ZUR STERILISATION VON KAPPEN
MACHINE DE STÉRILISATION COMPACTE DE CAPSULES

(43) Date of publication of application: 15.11.2023
(73) Proprietor: Sidel Participations, 76930 Octeville-sur-Mer (FR)
(72) Inventor: BORGESE, Rossana, 43126 Parma (IT)
(74) Representative: Sidel Group

(56) References cited:
- EP-A1- 3 670 404
- EP-A1- 3 991 757
- WO-A1-2022/090266

## Description

### TECHNICAL FIELD

The present invention relates to a sterilization machine for caps, in particular caps for receptacles, even more in particular caps for bottles.

### BACKGROUND ART

The sterilization of materials required for the packaging of products, such as food products, is of fundamental interest to guarantee the necessary shelf-life of the products packaged and, consequently, to ensure consumers' safety. This is even more important when the food products are packaged in aseptic conditions.

Filling of receptacles, such as containers, mays and bottles made of base components such as glass, plastic, aluminum, steel and composite materials, with any type of pourable food product, such as carbonated liquids (e.g., sparkling water, soft drinks and beer), non-carbonated liquids (including still water, fruit juices, tea, sports drinks, wine, milk, milk drinks, yoghurt drinks, flavoured water, etc.), emulsions and drinks containing pulp is known.

In general, before being filled with the pourable food product, the receptacles are sterilized in a receptacle sterilization device and are subsequently filled with the desired pourable food product in a filling machine.

After filling of the receptacles, typically, the respective openings of the receptacle through which filling takes place are closed by means of the application of respective caps.

Before application, the caps are sterilized in a respective sterilization machine. After sterilization, the sterile caps are fed to a capping machine, which also receives the filled receptacles.

A typical cap sterilization machine comprises a conveying device with a guide rail housed in an isolation tunnel. The caps are positioned in succession and in contact with one another on the guide rail and by means of a pusher element, such as an insertion star, the caps are inserted one at a time and placed in contact with the succession of caps already present within the guide rail. This leads to a thrust force being exerted on the succession of caps, which results in the advancement of the succession of caps.

A disadvantage of these known sterilization machines lies in the impossibility of adapting the related operating speed as a function of the working conditions of the capping machines.

A further problem of these known sterilization machines is that due to combination of high temperatures to which the caps are exposed and the contact between the caps, the caps are susceptible to deformations due to the forces acting within the succession of caps.

Therefore, with particular reference to EP 3 670 404 A1, which discloses a sterilization machine according to the preamble of claim 1, the Applicant has developed an alternative sterilization machine, which is based on a conveying device, which comprises a guide rail arranged inside an isolation tunnel and an endless rail, which carries a plurality of groups of moving carts. Each group of carts is operatively coupled to a respective pusher element, which is designed to interact with a respective group of a limited number of caps arranged on the guide rail in such a manner to advance the respective group.

In one embodiment, the endless rail is arranged under the isolation tunnel and each moving cart is provided with a first interaction member. Furthermore, the conveying device comprises a plurality of auxiliary carts arranged inside the isolation tunnel and coupled movably on a guide. Each auxiliary cart is associated with a respective cart and comprises a respective second interaction element configured to magnetically interact with the respective first interaction element in such a manner to transfer the advancement of the respective cart to an advancement of the auxiliary cart.

Furthermore, it should be noted that to guarantee the required hygienic condition within the isolation tunnel, the auxiliary carts are arranged in an auxiliary chamber which in turn is arranged inside the isolation tunnel. However, in this way, the mechanical complexity of the sterilization machine is increased.

Furthermore, it has been observed that the interaction between the auxiliary carts and the guide causes the formation of detritus, in particular inside the auxiliary chamber.

The object of the present invention is to produce a cap sterilization machine, which allows at least one of the aforementioned drawbacks to be overcome in a simple and economical manner.

The aforesaid object is achieved by the present invention, as it relates to a sterilization machine as defined in the independent claim 1. Alternative preferred embodiments are protected by the dependent claims.

### BRIEF DESCRIPTION OF THE DRAWINGS

For a better understanding of the present invention, a preferred embodiment thereof is described below, purely by way of a non-limiting example and with reference to the accompanying drawings, wherein:
- Figure 1 illustrates, schematically and in a top view, a sterilization machine for caps according to the present invention, with parts removed for clarity;
- Figure 2 illustrates, in a schematic side and sectional view, a portion of the sterilization machine of Figure 1, with parts removed for clarity;
- Figure 3 illustrates, in a schematic front and sectional view, a further detail of the sterilization machine of Figure 1, with parts removed for clarity; and
- Figure 4 illustrates, in a schematic view, an even other detail of the sterilization machine of Figure 1, with parts removed for clarity.

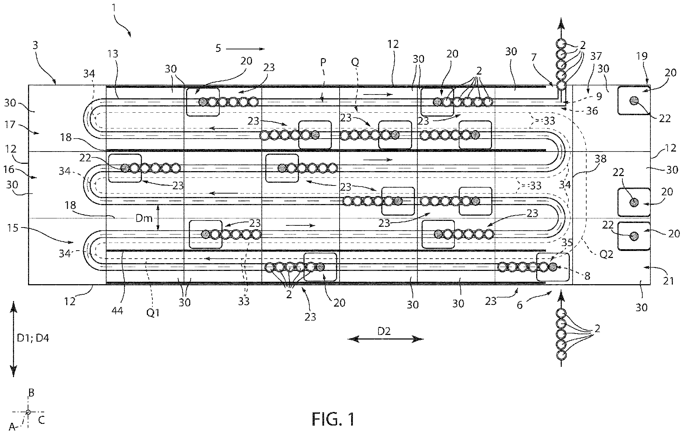

With reference to Figure 1, number 1 indicates as a whole a sterilization machine for the sterilization of caps 2.

In particular, sterilization machine 1 may be configured to sterilize caps 2 that may be applied to receptacles, such as bottles, containers or the like, containing a pourable food product.

The receptacles may be made of a thermoplastic polymer, as for example polyethylene terephthalate. Additionally or alternatively, the receptacles may also be made of a different material such as glass, a metal material, a composite material, a multi-layer material and the like.

In more detail, the pourable products may, for example, be carbonated liquids (such as sparkling water, soft drinks and beer), non-carbonated liquids (such as still water, fruit juices, wine, tea, milk, milk drinks, yoghurt drinks, flavored water), emulsions, suspensions, high viscosity liquids, emulsions and drinks containing pulp.

According to some embodiments, caps 2 may be made of a polymeric material, such as polyethylene, in particular high-density polyethylene.

Alternatively or additionally, caps 2 may vary in format (for example, in their extension - height, diameter, etc.) and/or in their type. For example, caps 2 may comprise an internal thread to be screwed onto receptacles In particular, caps 2 may be of the "sports cap" or "screw cap" type.

With particular reference to Figure 1, sterilization machine 1 may comprise an isolation tunnel 3 having or delimiting an inner space 4, in which the caps 2 are advanced along a conveying path P.

Moreover, isolation tunnel 3 may separate inner space 4 from an outer space 5. In particular, outer space 5 may define a hostile environment; i.e. an environment containing contaminants such as bacteria, microbes, powders, dust and the like.

Additionally, sterilization machine 1 may comprise a conditioning device configured to control the physical and/or chemical conditions within inner space 4. In particular, sterilization machine 1 may be configured such that inner space 4 may be aseptic and/or sterile. For example, the conditioning device may be configured to control the temperature, pressure, humidity, sterility and/or the chemical composition within inner space 4 and/or to control the flow of gases present in the inner space 4.

Preferably, the conditioning device may be configured to control the physical and/or chemical conditions in the inner space 4 locally; i.e., the physical and/or chemical conditions may vary in different portions of the inner space 4. In particular, as will be disclosed in further detail further below, isolation tunnel 3 may comprise a plurality of zones, which are configured to serve for varying purposes during the sterilization process and the conditions within these varying zones may differ.

Advantageously, the conditioning device may be configured to maintain an aseptic condition within inner space 4.

In more detail, isolation tunnel 3 may comprise an inlet 6 for caps 2 to be sterilized and an outlet 7 for the sterilized caps 2.

In particular, inlet 6 and outlet 7 may be arranged at opposite ends of isolation tunnel 3.

In more detail, conveying path P may extend between a start station 8 (being arranged substantially at inlet 6) and an end station 9 (being arranged substantially at outlet 7). In particular, in use, caps 2 advance from start station 8 to end station 9. More specifically, caps 2 are, in use, sterilized during their advancement along conveying path P (i.e., during their advancement from start station 8 to end station 9).

With particular reference to Figure 1, isolation tunnel 3 may comprise a plurality of walls that delimit inner space 4 and/or which may be arranged within inner space 4.

In more detail, this plurality of walls may comprise a lower wall 10 and an upper wall 11, in particular which may be distanced from one another along a vertical axis A. Additionally, upper wall 11 and lower wall 10 may be transversal to vertical axis A. Preferentially, vertical axis A may be operatively parallel to gravity and/or may be substantially vertical.

Additionally, the plurality of walls may also comprise a plurality of lateral walls 12.

In particular, isolation tunnel 3 may comprise at least two pairs of two lateral walls 12 so as to enclose inner space 4.

Moreover, the respective lateral walls 12 of a first pair of two lateral walls 12 may be distanced from one another along an axis B, in particular a first horizontal axis B. In particular, the respective two lateral walls 12 may be transversal to first horizontal axis B.

Advantageously, axis B may be operatively transversal to gravity.

Additionally, the respective lateral walls 12 of a second pair of two lateral walls 12 may be distanced from one another along an axis C, in particular a second horizontal axis C, preferentially second horizontal axis C being transversal and/or perpendicular to first horizontal axis B. In a preferred embodiment, first horizontal axis B is orthogonal to second horizontal axis C and vertical axis A, and vertical axis A is orthogonal to first horizontal axis B and second horizontal axis C. In particular, the respective two lateral walls 12 may be transversal to second horizontal axis C.

Advantageously, also second horizontal axis C may be operatively transversal to gravity.

According to some preferred non-limiting embodiments, sterilization machine 1 may comprise at least one guide rail 13 arranged within inner space 4 and configured to support caps 2 during their advancement along conveying path P. In particular, guide rail 13 determines the conveying path P.

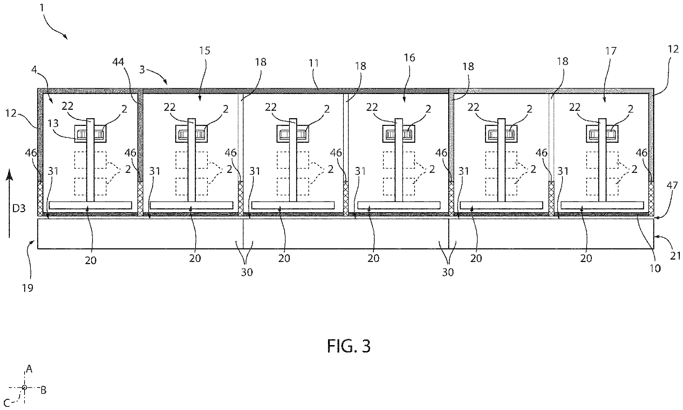

According to some possible non-limiting embodiments, sterilization machine 1 may comprise a plurality of guide rails 13 (see e.g. Figure 3). Each guide rail 13 is configured to support a respective format and/or type of caps. In use, caps 2 may be fed to and advanced along the respective guide rail 13, which may be adopted for the respective format and/or type of cap.

Moreover, each guide rail 13 may comprise at least an inlet section, in particular arranged substantially at start station 8, and an output section, in particular arranged substantially at end station 9, to respectively allow caps 2 to be sterilized to be fed to the respective guide rail 13 and the sterilized caps 2 to exit from the respective guide rail 13.

According to some preferred embodiments, isolation tunnel 3, in particular inner space 4, may comprise a plurality of zones defined in dependence of the operations to which the caps 3 are exposed while being conveyed through the respective zones.

According to some preferred non-limiting embodiments, isolation tunnel 3 may comprise at least:
- an injection zone 15;
- a contact and/or activation zone 16 being arranged downstream of injection zone 15 along conveying path P; and
- a drying zone 17 arranged downstream of contact and/or activation zone 16 along conveying path P.

In particular, in use, caps 2 are exposed to a sterilizing fluid when advancing within injection zone 15, in particular the sterilizing fluid deposits on caps 2.

Preferentially, in use, when caps 2 advance within contact and/or activation zone 16 the sterilizing fluid, in particular the sterilizing, which has deposited onto caps 2 during their advancement within injection zone 15, acts on caps 2.

Moreover, in use, when caps 2 advance within drying zone 19 the sterilizing fluid present on caps 2 evaporates from caps 2.

In more detail, sterilizing machine 1, in particular the conditioning device, may comprise a sterilizing unit configured to inject a sterilizing fluid (such as hydrogen peroxide and/or any other chemical sterilizing agent in gaseous, vapor and/or liquid form) into injection zone 15 of isolation tunnel 3, in particular into the respective portion of inner space 4. In particular, the sterilizing unit is configured to generate a cloud of the sterilizing fluid within injection zone 15 and such that the sterilizing fluid deposits, in use, on caps 2 during their advancement within injection zone 15.

Moreover, during advancement of caps 2 within contact and/or activation zone 16, the sterilizing agent acts on caps 2 so as to sterilize the same. Preferentially, the condition unit may comprise a temperature control unit configured to control temperatures within contact and/or activation zone 16, which guarantee an optimal activity of the sterilizing fluid.

Preferably, the conditioning device may also comprise a ventilation unit coupled to drying zone 17, in particular configured to ventilate drying zone 17.

According to some preferred non-limiting embodiments, isolation tunnel 3 may comprise a plurality of partition walls 18 arranged within inner space 4 and delimiting injection zone 15, contact and/or activation zone 16 and drying zone 17 within inner space 4.

Preferentially, partition walls 18 are parallel to one another.

According to some non-limiting embodiments, partition walls 18 may extend parallel to second horizontal axis C.

With particular reference to Figures 1 to 4, sterilization machine 1 may comprise a conveying device 19 configured to control advancement of caps 2 along conveying path P.

In more detail, conveying device 19 may comprise:
- a plurality of carts 20 positioned within inner space 4; and
- an actuation unit 21, in particular arranged within outer space 5, and configured to selectively advance carts 20 along an advancement path Q by means of the generation and control of an electromagnetic field, in particular along an endless or and/or annular advancement path Q.

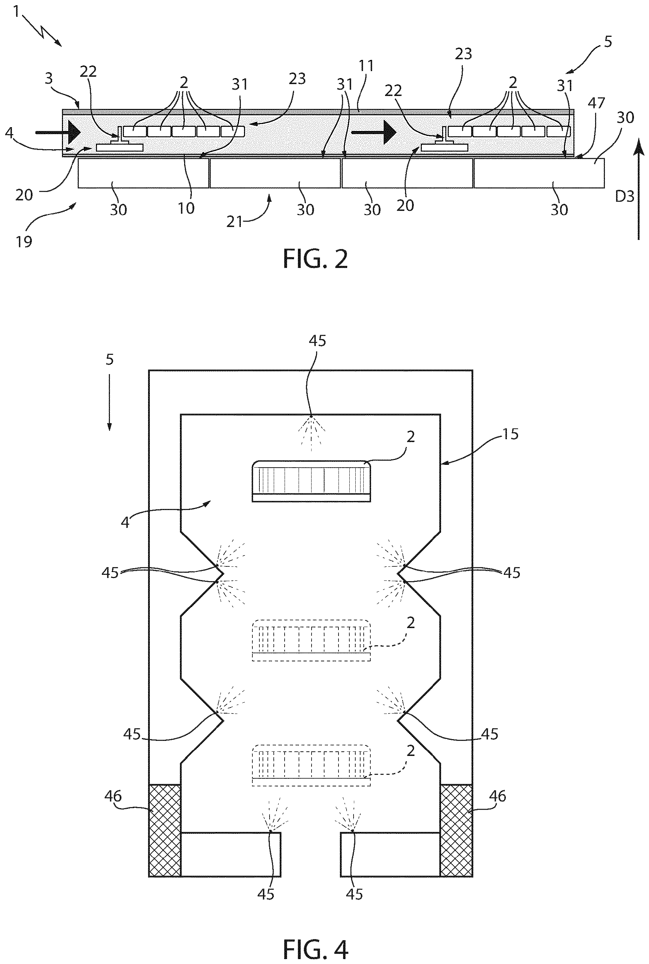

At least one wall of isolation tunnel 3 may be spatially interposed between actuation unit 21 and inner space 4. According to the specific embodiment shown (see e.g. Figures 2 and 3), the interposed wall could be lower wall 10. According to a non-shown embodiment, the interposed wall could be upper wall 11.

Preferentially, each cart 20 may comprise at least one pusher 22.

More specifically, for each cart 20, sterilizing machine 1 may be configured so that each pusher 22 interacts, in use, with and/or pushes a respective group 23 of caps 2, such that, by means of advancement of the cart 20 along at least one operative portion Q1 of advancement path Q, the group 23 advances along conveying path P.

According to some preferred non-limiting embodiments, the respective caps 2 of each group 23 may be arranged in succession to one another. In the specific case illustrated in Figures 1 and 2, each group 23 may comprise five caps 2. Preferably, each group 23 may comprise a number of caps whose value may fall between 1 and 20 or between 1 and 15.

In the specific case of Figures 1 and 2, each group 23 comprises the same number of caps 2. However, the number of caps 2 of the groups 23 may, according to some embodiments, vary from one another.

In more detail, in use, by means of the advancement of the carts 20 along operative portion Q1, an advancement of pushers 22, which in turn push the respective groups 23 along the conveying path P, is obtained.

Moreover, for each cart 20, sterilizing machine 1 may be configured such that, by means of a pushing action exerted by the respective pusher 22 on the respective group 23, the advancement of cart 20 along operative portion Q1 corresponds to the advancement of the respective group 23 along conveying path P.

Additionally, for each cart 20, sterilizing machine 1 may be configured so that, by means of this pushing action, the advancement of the cart 20 along this operative portion Q1 causes the advancement of the respective group 23 along the conveying path P.

According to some preferred non-limiting embodiments, actuation unit 21 could be defined by and/or may comprise a planar motor.

With particular reference to Figure 1, actuation unit 21 may comprise a plurality coils and a controller configured to selectively (electrically) supply the coils so as to control, in particular to locally control, the electromagnetic field.

In particular, the controller may be configured such to selectively control advancement of carts 20 by selectively controlling the coils.

Preferentially, actuation unit 21 may comprise a plurality of planar actuation modules 30, each one having a plurality of the coils. Each actuation module corresponds to a respective planar motor tile.

Moreover, planar actuation modules 30 may be arranged next to one another along a first horizontal direction D1, which is in particular parallel to first horizontal axis B, and next to one another along a second horizontal direction D2 which is transversal, in particular perpendicular, to first horizontal direction D1. The second horizontal direction D2 can be parallel to second horizontal axis C.

In particular, each planar actuation module 30 may comprise a first face 31 facing isolation tunnel 3, in particular lower wall 10 (as shown in the specific disclosed embodiment) or upper wall 11 (according to a non-shown embodiment).

Moreover, the plurality of first faces 31 may define a common surface area of the actuation unit 21, in particular the common area extending along first horizontal direction D1 and second horizontal direction D2 and facing isolation tunnel 3. In particular, in this way, it is possible to locally control the electrical field within an area extending in two dimensions.

Preferentially, isolation channel 3 may be superimposed on the common surface area.

In further detail, lower wall 10 or upper wall 11 may be interposed between planar actuation modules 30 and carts 20.

In further detail, for each cart 20, actuation unit 21 may be configured in such a manner to control by means of magnetic levitation a first position component of cart 20, in particular independently from the other carts 20. This first position component may be along a vertical direction D3, in particular being perpendicular to first horizontal direction D1 and second horizontal direction D2 or perpendicular to the common area.

In more detail, the respective first position component is with respect to actuation unit 21, in particular planar actuation modules 30, and/or to isolation tunnel 3.

Preferentially, vertical direction D3 may be locally transversal and/or orthogonal to advancement path Q. Even more preferentially, vertical direction D3 may lie locally on a plane that is transversal and/or orthogonal to advancement path Q.

According to some preferred non-limiting embodiments, actuation unit 21 may be configured to carry out the selective control of the respective first position components of carts 20 during their advancement along advancement path Q.

In particular, with actuation unit 21 being defined by and/or comprising a planar motor is particularly suitable to control the first position components.

According to some preferred non-limiting embodiments, for each cart 20, actuation unit 21 may be configured in such a manner to control, by means of control, in particular a local control, of the electromagnetic field and independently from the other carts 20, also a second position component of cart 20 with respect to actuation unit 21 and/or to isolation channel 3. Second position component may be along a fourth direction D4, in particular parallel to first horizontal direction D1.

Preferentially, fourth direction D4 may be locally transversal and/or orthogonal to advancement path Q and to vertical direction D3.

In particular, actuation unit 21 may be configured to selectively carry out control of the second position component during advancement of each cart 20 along advancement path Q.

As may be seen in Figures 2 and 3, vertical direction D3 may be parallel to vertical axis A. Additionally, fourth direction D4 may be parallel to first horizontal axis B.

For each cart 20, the respective first position component may be considered as an elevation or height of the cart 20 within inner space 4. In particular, the elevation or height can be considered correlated to a vertical distance with respect to actuation unit.

More specifically, the respective vertical distance between each cart 20 and actuation unit 21 may be defined by the vertical gap between each cart 20 and the common surface area and/or one respective face 31, in particular along an axis normal to the respective face 31 and/or the common surface area.

Preferably, for each cart 20, actuation unit 21 may be configured to control the respective vertical distance such that the value of this distance falls within the interval from 0.5 mm to 15.0 mm, in particular from 0.5 mm to 10.0 mm, or from 0.5 mm to 5 mm. In this way the vertical distance is sufficient to avoid risks of interference due to disturbances between cart 20 and isolation tunnel 3, in particular lower wall 10 or upper wall 11, while at the same time also ensuring sufficient control of actuation unit 21 on the advancement of each cart 20.

In further detail, for each cart 20, the second position component could be considered a lateral position, in particular with regard to advancement path Q, of cart 20 in inner space 4. In this way, actuation unit 21 may be configured to generate and control (regulate) the electromagnetic field that interacts selectively by means of electromagnetic forces with carts 20, in such a manner to advance the carts 20 along the advancement path Q and simultaneously control the transversal position of the carts 20 along or on a plane transversal to the advancement path Q.

In particular, due to the control of the elevation and/or of the lateral position by means of electromagnetic effect and/or by means of magnetic levitation, each cart 20 may have a simpler mechanical configuration, which reduces the risk of contamination.

In more detail, for each cart 20, actuation unit 21 may be configured in such a manner to control the first position component maintaining cart 20 distanced from lower wall 10 and/or from upper wall 11 of isolation tunnel 3, independently from the other carts 20. Preferentially, actuation unit 21 may be configured to maintain each cart 20 distanced from lower wall 10 and/or from upper wall 11, independently from the other carts 20 and acting against gravity.

In this way, each cart 20 may be controlled in a very precise manner, so as to reduce the risk of interference between cart 20 and upper wall 11 and/or between cart 20 and lower wall 10.

Moreover, the extension of isolation tunnel 3 along vertical axis A may be reduced due to the fact that the first position component may be controlled in a precise manner.

It should be noted that, without the control of the first position component, the carts 20 would fall as a result of gravity towards lower wall 10, and/or could contact lower wall 10 and/or upper wall 11 as a result of disturbances.

Sterilization machine 1 may be configured to allow a user to set up in advance a desired value of this first position component as a function of the format and/or type of cap. For each cart 20, the actuation unit 21 may be configured to control in advance the first position component of each cart 20 as a function of the desired value.

In this way, the user may intuitively and conveniently adapt sterilization machine 1 to the specific format and/or type of cap, so as to improve the flexibility of sterilization machine 1.

The control of the first position component is carried out in such a manner to pursue and/or maintain this set desired value of the first position component.

In this way, the actuation unit 21 is configured to generate and control (adjust) the electromagnetic field that selectively interacts by means of electromagnetic forces with carts 20, in such a manner to advance carts 20 along advancement path Q and control the transversal position of the carts 20 along a plane transversal to the advancement path Q.

In particular, actuation unit 21 may be configured to advance carts 20 independently from one another by means of the generation and the control of the electromagnetic field. Even more in particular, actuation unit 21 may be configured to accelerate and/or decelerate carts 20 independently from one another and/or modify a positioning of carts 20 independently from one another by means of the generation and the control of the electromagnetic field.

In more detail, actuation unit 21 may be configured to control the respective second position component of each cart 20 by maintaining cart 20 distanced from lateral walls 12 of isolation tunnel 3.

Actuation unit 21 may be configured to maintain each cart 20 distanced from the lateral walls 12 and/or partition walls 18 independently from the other carts 20.

It should be noted that, in the absence of the control of the second position component, carts 20 could contact one of the lateral walls 12 and/or partition walls 18 due to disturbances.

In particular, during the advancement, carts 20 advance without being in contact with any portions of isolation tunnel 3.

Therefore, by means of the control of the first position component of the carts 20 along vertical direction D3, actuation unit 21 may be configured to vertically control carts 20 during their advancement, and consequently a respective vertical distance between carts 20 and lower wall 10 and/or between carts 20 and upper wall 11. Moreover, by means of the control of the carts 20 along fourth direction D4, actuation unit 21 may be configured to control carts 20 laterally or horizontally during their advancement.

Sterilizing machine 1 may be void of any mechanical guide for guiding by means of mechanical contact the advancement of carts 20 along advancement path Q. In this way, advancement path Q may be defined solely by the control of the electromagnetic field, so as to further simplify the mechanics of sterilization machine 1, further limiting the risk of undesirable contaminations.

According to some preferred non-limiting embodiments, one or more partition walls 18 may comprise at least one passage configured to allow for the passage of carts 20. E.g. the respective passage of the partition wall 18 delimiting injection zone 15 from contact and/or activation zone 16 allows passage of carts 20 from injection zone 15 into contact and/or activation zone 16 or the respective passage of the partition wall 18 delimiting contact and/or activation zone 16 from drying zone 17 allows passage of carts 20 from contact and/or activation zone 16 into drying zone 17.

According to some preferred non-limiting embodiment, each cart 20 may comprise and/or consist of a magnetic or ferromagnetic portion configured to interact with the electromagnetic field and such to control advancement of carts 20 along advancement path Q.

With particular reference to Figure 1, operative portion Q1 may be serpentine shaped.

In this way, it is possible to optimize the space-requirements of sterilizing machine 1 and to optimize the use of actuation unit 21.

Preferentially, according to the shape of operative portion Q1, also conveying path P and/or guide rail(s) 13 may present a serpentine shape.

Please note that in the following, further details about operative portion Q1 are disclosed. It should be noted that the described details apply by analogy also to conveying path P and/or guide rail(s) 13.

In more detail, operative portion Q1 may comprise:
- a plurality of linear sections 33, in particular at least four, even more particular at least six; and
- a plurality of curved sections 34, in particular at least three, even more particular at least five.

Additionally, each curved section 34 may connect two respective linear sections 33 with one another.

In particular, linear sections 33 may be successively arranged with respect to one another and along operative portion Q1.

In further detail, each curved section 34 may be interposed between one respective upstream linear section 33 and one respective downstream linear section 33. In particular, the terms upstream and downstream are defined with respect to operative portion Q1.

In other words, each downstream linear section 33 may be arranged downstream from the respective upstream linear section 33 along operative portion Q1.

Moreover, each curved section 34 may connect the respective upstream linear section 33 with the respective downstream linear section 33.

It should be noted that some of the linear sections 33 may be an upstream linear section 33 with respect to a first curved section 34 and may be a downstream linear section 33 with respect to a second curved section 34.

According to some preferred non-limiting embodiments, sterilization machine 1 may be configured such that, in use, carts 20 (and accordingly also the respective groups 23 of caps 2) may advance according to a respective first advancement direction along upstream linear section 33 and according to a respective second advancement direction along the respective downstream linear section 33, the second advancement direction being opposite to the respective first advancement direction. In this way the length of distance which can be covered by the caps with a same row of planar motor tiles or modules 30 is increased, leading to a reduction of costs. This is obtained by means of the above-mentioned serpentine shape.

With particular reference to Figure 1, each linear section 33 may be parallel to the other linear sections 33.

Advantageously, at least some, in particular all, of linear sections 33 may (substantially) have the same length.

Additionally, linear sections 33 may be spaced apart along a main direction Dₘ normal to linear sections 33 themselves. In particular, main direction Dₘ may be parallel to axis B and/or first horizontal direction D1 and/or fourth direction D4.

In particular, the end portions of isolation tunnel 3 having inlet 6 and outlet 7 may be spaced apart along main direction Dₘ, too.

According to the specific embodiment shown, at least two respective linear sections 33 lie within each one of injection zone 15, contact and/or activation zone 16 and drying zone 17. This guarantees that caps 2 remain in the respective zones for the required times.

According to some of possible embodiments, some curved sections 34 lie within one of injection zone 15, contact and/or activation zone 16 and drying zone 17.

Additionally or alternatively, one curved section 34 may lie partially within injection zone 15 and partially within contact and/or activation zone 16 and/or another curved section 34 may lie partially within contact and/or activation zone 16 and partially within drying zone 17.

According to some preferred non-limiting embodiments, actuation unit 21 may be configured to control the electromagnetic field such as to modify and/or control the roll inclination and/or the pitching inclination of each cart 20 during the advancement, in particular along at least curved sections 34. In this way, the effects of the centrifugal force may be offset.

In further detail, operative portion Q1 may extend from an engagement station 35 at which each pusher 22 starts, in use, to interact with the respective group 23 of caps 2 to a release station 36 at which each pusher 22 is released, in use, from the respective group 23 of caps 2. In particular, engagement station 35 and release station 36 may be adjacent to start station 8 and end station 9, respectively.

Preferentially, engagement station 35 may lie within injection zone 15 and release station 36 may lie within drying zone 17.

According to some preferred non-limiting embodiments, advancement path Q may also comprise a return portion Q2, in particular so as to bring carts 20 back onto operative portion Q1. In particular, operative portion Q1 and return portion Q2 may define the endless shape of advancement path Q, thereby allowing a cyclic operation of carts 20.

In more detail, while advancing along operative portion Q1 carts 20, in particular the respective pusher 22, may interact with the respective group 23 of caps 2 and while advancing, in use, along return portion Q2 carts 22, in particular the respective pusher 22, is free of any interaction with caps 2.

More specifically, return portion Q2 may extend between release station 36 and engagement station 35.

According to some preferred non-limiting embodiments, isolation tunnel 3 may also comprise an auxiliary zone 37. In particular, auxiliary zone 37 may be adjacent to and laterally displaced from injection zone 15, contact and/or activation zone 16 and drying zone 17.

Moreover, one partition wall 38 of isolation tunnel 3 may delimit auxiliary zone 37 from injection zone 15, contact and/or activation zone 16 and drying zone 17. Additionally, partition wall 38 may comprise a first aperture allowing for transfer of carts 20 from drying zone 17 into auxiliary zone 37 and a second aperture allowing for transfer from auxiliary zone 37 into injection zone 15.

According to some preferred non-limiting embodiments, a central section of return portion Q2 lies within auxiliary zone 37.

Moreover, during the advancement of the carts 20 along the advancement path Q, the pushers 22 pass through the start station 8 and the end station 9. In particular, the end station 9 may be arranged downstream of the start station 8 along the advancement path Q.

In further detail, for each cart 20, the respective pusher 22 comes, in use, cyclically into contact with a respective group 23 at the start station 8 (i.e. with the respective cart 20 being at engagement station 35) and detaches cyclically from the respective group 23 at end station 9 (i.e. with the respective cart 22 being at release station 36). During the advancement of each pusher 22 from start station 8 to the end station 9, the pusher 22 is in contact with the group 23. During the advancement of each pusher 22 from end station 9 to start station 8, the pusher 22 is not in contact with any cap 2.

Moreover, actuation unit 21 may be configured to advance carts 20 along advancement path Q continuously so that the respective pushers 22 advance through start station 8 and end station 9 continuously; i.e., each time each pusher 22 passes through start station 8, pusher 22 comes into contact with a respective new group 23 to push the respective new group 23 towards end station 9.

According to some possible embodiments, isolation tunnel 3 may also comprise an auxiliary wall 44 arranged within injection zone 15.

With particular reference to Figure 4, the sterilization unit may comprise:
- one or more injection orifices 45 and/or injection nozzles for injecting the sterilizing fluid into injection zone 15; and
- a feeding conduit for feeding the sterilizing fluid to injection orifices 45 and/or the injection nozzles.

According to some preferred non-limiting, the feeding conduit may be arranged within one or more respective walls of isolation tunnel 3 and/or injection orifices 45 and/or the injection nozzles may be connected to one or more respective walls of isolation tunnel 3. In this way the sterilization fluid is fed through the walls of the tunnel 3, leading to a more compact machine.

For example, the lateral wall 12 delimiting injection zone 15 and/or the partition wall 18 delimiting injection zone 15 and/or auxiliary wall 44 may comprise a respective feeding conduit connected to respective injection orifices 45 and/or injection nozzles.

According to some preferred non-limiting embodiments, sterilizing machine 1, in particular the conditioning unit, may comprise a plurality of heating elements arranged within respective walls of isolation tunnel 3. For example, the heating elements may be arranged within one or more lateral walls 12 and/or partition walls 38 and/or partition wall 38 and/or auxiliary wall 44. In this way at least one section of the inner space 4 can be heated through the walls of the tunnel 3, leading to a more compact machine. The heating elements can comprise for example an electrical heating system, which can comprise a resistor, or a heating induction system.

In particular, the heating elements may be configured to heat portions of the respective walls so as to heat respective sections of inner space 4.

Additionally, each wall carrying respective heating elements may also comprise a thermal isolation insert 46 configured to thermally protect or isolate actuation unit 21, in particular planar actuation modules 30, from the one or more heating elements and/or from the respective heated walls. Preferentially, each thermal isolation insert 46 may be exchangeable.

According to some preferred non-limiting embodiment, sterilizing machine 1 may comprise a cooling device for cooling actuation unit 21.

Preferentially, the cooling device may be configured to generate a cooling flow in and/or through an interspace 47 interposed between isolation tunnel 3 and actuation unit 21. In particular, the cooling flow may comprise a flow of cooling air and/or a flow of cooling water and/or a flow of any other cooling fluid.

In this way, damage or excessive wear of actuation unit 21 due to the heat released during the sterilization process may be avoided.

According to some non-limiting embodiments, actuation unit 21 may be configured to modulate an advancement speed of the carts 20 by means of the control of the electromagnetic field, in particular as a function of the portion of the advancement path along which carts 20 advance at a specific time. For example, the speed may be controlled as a function of the fact that carts 20 advance within injection zone 15, in contact and/or activation zone 16 or in drying zone 17.

In use, sterilization machine 1 sterilizes caps 2 while they are being conveyed within inner space 4 and along conveying path P.

In more detail, the operation of the sterilization machine 1 comprises at least the steps of:
- advancing carts 20 along advancement path Q; and
- pushing groups 23 of caps 2 along conveying path P due to the advancement of carts 20 along operative portion Q1 and the contact of the respective pusher 22 with the respective groups 23.

Additionally, the operation of the sterilization machine 1 may further comprise the steps of:
- feeding caps 2 onto the respective guide rail 13 at start station 8; and/or
- unloading caps 2 from the respective guide rail 13 at end station 9; and/or
- injecting the sterilizing fluid into injection zone 15; and/or
- ventilating drying zone 17.

From an examination of the features of sterilization machines 1 according to the present invention the advantages that may be obtained therewith are evident.

In particular, sterilization machines 1 comes along with optimized space requirements.

Additionally, planar actuation modules 30 can be optimally used. Their two-dimensional extension can be used to allow for advancement into a respective first advancement direction and a respective second advancement direction opposed to the first advancement direction. This allows a save of costs related to the modules 30, and is in particular connected to the serpentine shape of the operative portion Q1.

Additionally, sterilization machine is void of a mechanical guide for carts 20. This reduces the complexity and allows the formation of detritus caused by contact between the carts 20 and a mechanical guide to be avoided.

A further advantage lies in the fact that the electromagnetic field acts directly on carts 20 present in the inner space 4, which allows a further reduction of the complexity.

Finally, it is clear that modifications and variants may be made to the sterilization machine described and illustrated without departing from the scope of protection defined by the claims.

Sterilization machine 1 may comprise a plurality of guide rails, which may be arranged on top of one another as indicated e.g. in Figure 3 or which may be laterally displaced from one another, allowing an increase in flexibility with respect to the type or format of caps 2.

## Claims

1. Sterilization machine (1) for the sterilization of caps (2) comprising at least:
- an isolation tunnel (3) having an inner space (4), the machine (1) being configured such that the caps (2) are advanced along a conveying path (P) placed within the inner space (4), said isolation tunnel (3) separating the inner space (4) from an outer space (5);
- a plurality of carts (20) positioned within the inner space (4); and
- an actuation unit (21) arranged in the outer space (5) and configured to advance the carts (20) along an advancement path (Q) by means of the generation of an electromagnetic field;
wherein each cart (20) comprises at least one pusher (22) configured to interact with a group (23) of caps (2) having one or more caps (2) and such that the advancement of each cart (20) along an operative portion (Q1) of the advancement path (Q) corresponds to the advancement of the respective group (23) along the conveying path (P), said at least one operative portion (Q1) being arranged within the inner space (4);
wherein, for each cart (20), the actuation unit (21) is configured to control the advancement of the cart (20) along the advancement path (Q), by means of control of the electromagnetic field and independently from the other carts (20);
**characterized in that**,
for each cart (20) advancing independently from the other carts (20), the actuation unit (21) is configured to control by means of magnetic levitation a first position component of the cart (20) with respect to the actuation unit (21), said first position component being along a vertical direction (D3), said vertical direction (D3) being transversal to the advancement path (Q) ;
wherein the operative portion (Q1) is serpentine shaped.

2. Sterilization machine according to claim 1, wherein the operative portion (Q1) comprises a plurality of linear sections (33) and a plurality of curved sections (34); wherein each curved section (34) connects two respective linear sections (33) with one another.

3. Sterilization machine according to claim 2, wherein each curved section (34) connects a respective upstream linear section (33) and a respective downstream linear section (33) to one another, wherein the downstream linear section (33) is arranged downstream from the respective upstream linear section (33) along the operative portion (Q1);
wherein the sterilization machine (1) is configured such that the carts (22) are advanced according to a respective first advancement direction along the respective upstream linear section (33) and according to a respective second advancement direction along the respective downstream linear section (33), the first advancement direction being opposite to the second advancement direction.

4. Sterilization machine according to claim 2 or 3, wherein each linear section (33) is parallel to the other linear sections (33).

5. Sterilization machine according to any one of claims 2 to 4, wherein at least some, in particular all, of the linear sections (33) have the same length.

6. Sterilization machine according to any one of claims 2 to 5, wherein the linear sections (33) are spaced apart along a main direction (Dₘ) normal to the linear sections (33) themselves.

7. Sterilization machine according to any one of claims 2 to 6, wherein the operative portion (Q1) comprises four or more linear sections (33).

8. Sterilization machine according to any one of the preceding claims, wherein the isolation tunnel (3) comprises an injection zone (15), a contact and/or activation zone (16) being arranged downstream of the injection zone (15) along the conveying path (P) and a drying zone (17) arranged downstream of the contact zone (16) along the conveying path (P);
the machine being configured so that the caps (2) are exposed to a sterilizing fluid when advancing within the injection zone (15);
the machine being configured so that, when the caps (2) advance within the contact zone (16), the sterilizing fluid acts on the caps (2); and
the machine being configured so that, when the caps (2) advance within the drying zone (19), the sterilizing fluid present on the caps (2) evaporates from the caps (2) .

9. Sterilizing machine according to claim 8 and according to any one of claims 2 to 7, wherein at least two respective linear sections (33) of the operative portion (Q1) lie within each one of the injection zone (15), the contact zone (16) and the drying zone (17).

10. Sterilizing machine according to claim 8 or 9, wherein the isolation tunnel (3) comprises a plurality of walls (10, 11, 12, 18, 34, 44) delimiting and/or being arranged within the inner space (4) ;
wherein the sterilizing machine (1) comprises a sterilizing unit configured to inject a sterilizing fluid into the injection zone (15) of the isolation tunnel (3);
wherein the injection zone (15) is delimited by one of more walls (10, 11, 12, 18, 34, 44);
wherein the sterilization unit (1) comprises one or more injection orifices (45) and/or one or more injection nozzles for injecting the sterilizing fluid into the injection zone (15) and a feeding conduit for feeding the sterilizing fluid to the injection orifices (45) and/or the injection nozzles;
wherein the feeding conduit is arranged within one or more respective walls (10, 11, 12, 18, 34, 44).

11. Sterilizing machine according to claim 10, wherein one or more injection orifices (45) and/or one or more injection nozzles are connected to or supported by one or more respective walls (10, 11, 12, 18, 34, 44).

12. Sterilization machine according to any one of the preceding claims, wherein the operative portion (Q1) extends from an engagement station (35) at which each pusher (22) starts, in use, to interact with the respective group (23) of caps (2) to a release station (36) at which each pusher (22) is released, in use, from the respective group (23) of caps (2);
wherein the actuation unit (21) is configured to advance the carts (20) along the operative portion (Q1) of the advancement path (Q) and along a return portion (Q2) of the advancement path (Q), the return portion (Q2) extending between the release station (36) and the engagement station (35).

13. Sterilizing machine according to claim 10 or 11,
wherein the sterilizing machine (1) comprises a plurality of heating elements arranged within respective walls (10, 11, 12, 18, 34, 44);
wherein one or more of the walls (10, 11, 12, 18, 34, 44) comprises a thermal isolation insert (46) configured to thermally isolate or protect the actuation unit (21) from the one or more heating elements.

14. Sterilizing machine according to claim 13, wherein each thermal isolation insert (46) is exchangeable or replaceable.

15. Sterilizing machine according to any one of the preceding claims, wherein the actuation unit (21) is defined by and/or comprises a planar motor.

## Patentansprüche

1. Sterilisationsmaschine (1) zur Sterilisation von Deckeln (2), zumindest Folgendes umfassend:
- einen Isolationstunnel (3) mit einem Innenraum (4), wobei die Maschine (1) derart ausgelegt ist, dass die Deckel (2) entlang einer Förderstrecke (P) befördert werden, die innerhalb des Innenraums (4) platziert ist, wobei der Isolationstunnel (3) den Innenraum (4) vom Außenraum (5) trennt;
- mehrere Wagen (20), die innerhalb des Innenraums (4) positioniert sind; und
- eine Betätigungseinheit (21), die im Außenraum (5) angeordnet und dazu ausgelegt ist, die Wagen (20) mittels der Erzeugung eines elektromagnetischen Felds entlang einer Vorwärtsbewegungsstrecke (Q) vorwärts zu bewegen;
wobei jeder Wagen (20) mindestens einen Schieber (22) umfasst, der dazu ausgelegt ist, mit einer Gruppe (23) von Deckeln (2), die einen oder mehrere Deckel (2) aufweist, zusammenzuwirken, und sodass die Vorwärtsbewegung jedes Wagens (20) entlang eines Betriebsabschnitts (Q1) der Vorwärtsbewegungsstrecke (Q) der Vorwärtsbewegung der jeweiligen Gruppe (23) entlang der Förderstrecke (P) entspricht, wobei der mindestens eine Betriebsabschnitt (Q1) innerhalb des Innenraums (4) angeordnet ist;
wobei die Betätigungseinheit (21) für jeden Wagen (20) dazu ausgelegt ist, die Vorwärtsbewegung des Wagens (20) entlang der Vorwärtsbewegungsstrecke (Q) mittels der Steuerung des elektromagnetischen Felds und unabhängig von den anderen Wagen (20) zu steuern;
**dadurch gekennzeichnet, dass** die Betätigungseinheit (21) für jeden Wagen (20), der sich unabhängig von den anderen Wagen (20) vorwärtsbewegt, dazu ausgelegt ist, mittels magnetischen Schwebens eine erste Positionskomponente des Wagens (20) in Bezug zur Betätigungseinheit (21) zu steuern, wobei die erste Positionskomponente entlang einer vertikalen Richtung (D3) verläuft, wobei die vertikale Richtung (D3) quer zur Vorwärtsbewegungsstrecke (Q) verläuft;
wobei der Betriebsabschnitt (Q1) serpentinenförmig ist.

2. Sterilisationsmaschine nach Anspruch 1, wobei der Betriebsabschnitt (Q1) mehrere lineare Teilabschnitte (33) und mehrere gekrümmte Teilabschnitte (34) umfasst; wobei jeder gekrümmte Teilabschnitt (34) zwei jeweilige lineare Teilabschnitte (33) miteinander verbindet.

3. Sterilisationsmaschine nach Anspruch 2, wobei jeder gekrümmte Teilabschnitt (34) einen jeweiligen vorgelagerten linearen Teilabschnitt (33) und einen jeweiligen nachgelagerten linearen Teilabschnitt (33) miteinander verbindet, wobei der nachgelagerte lineare Teilabschnitt (33) entlang des Betriebsabschnitts (Q1) dem jeweiligen vorgelagerten linearen Teilabschnitt (33) nachgelagert angeordnet ist;
wobei die Sterilisationsmaschine (1) derart ausgelegt ist, dass die Wagen (22) entsprechend einer jeweiligen ersten Vorwärtsbewegungsrichtung entlang des jeweiligen vorgelagerten linearen Teilabschnitts (33) und entsprechend einer jeweiligen zweiten Vorwärtsbewegungsrichtung entlang des jeweiligen nachgelagerten linearen Teilabschnitts (33) vorwärtsbewegt werden, wobei die erste Vorwärtsbewegungsrichtung der zweiten Vorwärtsbewegungsrichtung entgegengesetzt ist.

4. Sterilisationsmaschine nach Anspruch 2 oder 3, wobei jeder lineare Teilabschnitt (33) parallel zu den anderen linearen Teilabschnitten (33) verläuft.

5. Sterilisationsmaschine nach einem der Ansprüche 2 bis 4, wobei zumindest einige der, insbesondere alle, linearen Teilabschnitte (33) dieselbe Länge aufweisen.

6. Sterilisationsmaschine nach einem der Ansprüche 2 bis 5, wobei die linearen Teilabschnitte (33) entlang einer Hauptrichtung (Dₘ) normal zu den linearen Teilabschnitten (33) selbst beabstandet sind.

7. Sterilisationsmaschine nach einem der Ansprüche 2 bis 6, wobei der Betriebsabschnitt (Q1) vier oder mehrere lineare Teilabschnitte (33) umfasst.

8. Sterilisationsmaschine nach einem der vorstehenden Ansprüche, wobei der Isolationstunnel (3) einen Einspritzbereich (15), einen dem Einspritzbereich (15) entlang der Förderstrecke (P) nachgelagert angeordneten Kontakt- und/oder Aktivierungsbereich (16) und einen dem Kontaktbereich (16) entlang der Förderstrecke (P) nachgelagert angeordneten Trockenbereich (17) umfasst;
wobei die Maschine derart ausgelegt ist, dass die Deckel (2) einem Sterilisationsfluid ausgesetzt sind, wenn sie sich innerhalb des Einspritzbereichs (15) vorwärtsbewegen;
wobei die Maschine derart ausgelegt ist, dass das Sterilisierungsfluid auf die Deckel (2) wirkt, wenn sich die Deckel (2) innerhalb des Kontaktbereichs (16) vorwärtsbewegen; und
wobei die Maschine derart ausgelegt ist, dass das auf den Deckeln (2) vorhandene Sterilisierungsfluid von den Deckeln (2) verdampft, wenn sich die Deckel (2) innerhalb des Trockenbereichs (19) vorwärtsbewegen.

9. Sterilisationsmaschine nach Anspruch 8 und nach einem der Ansprüche 2 bis 7, wobei zumindest zwei jeweilige lineare Teilabschnitte (33) des Betriebsabschnitts (Q1) jeweils innerhalb des Einspritzbereichs (15), des Kontaktbereichs (16) und des Trockenbereichs (17) liegen.

10. Sterilisationsmaschine nach Anspruch 8 oder 9, wobei der Isolationstunnel (3) mehrere Wände (10, 11, 12, 18, 34, 44) umfasst, die den Innenraum (4) begrenzen und/oder darin angeordnet sind;
wobei die Sterilisationsmaschine (1) eine Sterilisationseinheit umfasst, die dazu ausgelegt ist, ein Sterilisationsfluid in den Einspritzbereich (15) des Isolationstunnels (3) einzuspritzen;
wobei der Einspritzbereich (15) durch eine oder mehrere Wände (10, 11, 12, 18, 34, 44) begrenzt ist;
wobei die Sterilisationseinheit (1) eine oder mehrere Einspritzöffnungen (45) und/oder eine oder mehrere Einspritzdüsen zum Einspritzen des Sterilisationsfluids in den Einspritzbereich (15) und eine Zuführleitung zum Zuführen des Sterilisationsfluids in die Einspritzöffnungen (45) und/oder die Einspritzdüsen umfasst;
wobei die Zuführleitung innerhalb einer oder mehreren jeweiligen Wänden (10, 11, 12, 18, 34, 44) angeordnet ist.

11. Sterilisationsmaschine nach Anspruch 10, wobei eine oder mehrere Einspritzöffnungen (45) und/oder eine oder mehrere Einspritzdüsen mit einer oder mehreren jeweiligen Wänden (10, 11, 12, 18, 34, 44) verbunden sind oder davon getragen werden.

12. Sterilisationsmaschine nach einem der vorstehenden Ansprüche, wobei sich der Betriebsabschnitt (Q1) von einer Eingriffsstation (35), an welcher jeder Schieber (22) bei der Verwendung beginnt, mit der jeweiligen Gruppe (23) Deckel (2) zusammenzuwirken, zu einer Lösestation (36), an der jeder Schieber (22) bei der Verwendung von der jeweiligen Gruppe (23) Deckeln (2) gelöst wird, erstreckt;
wobei die Betätigungseinheit (21) dazu ausgelegt ist, die Wagen (20) entlang des Betriebsabschnitts (Q1) der Vorwärtsbewegungsstrecke (Q) und entlang eines Rückführungsabschnitts (Q2) der Vorwärtsbewegungsstrecke (Q) vorwärts zu bewegen, wobei sich der Rückführungsabschnitt (Q2) zwischen der Lösestation (36) und der Eingriffsstation (35) erstreckt.

13. Sterilisationsmaschine nach Anspruch 10 oder 11,
wobei die Sterilisationsmaschine (1) mehrere Heizelemente umfasst, die innerhalb jeweiliger Wände (10, 11, 12, 18, 34, 44) angeordnet sind;
wobei eine oder mehrere der Wände (10, 11, 12, 18, 34, 44) einen Wärmeisolierungseinsatz (46) umfassen, der dazu ausgelegt ist, die Betätigungseinheit (21) von dem einen oder den mehreren Heizelementen zu wärmeisolieren oder zu schützen.

14. Sterilisationsmaschine nach Anspruch 13,
wobei jeder Wärmeisolierungseinsatz (46) austauschbar oder auswechselbar ist.

15. Sterilisationsmaschine nach einem der vorstehenden Ansprüche, wobei die Betätigungseinheit (21) durch einen Planarmotor definiert ist und/oder diesen umfasst.

## Revendications

1. Machine de stérilisation (1) pour la stérilisation de bouchons (2) comprenant au moins :
- un tunnel d'isolation (3) comportant un espace intérieur (4), la machine (1) étant conçue de telle sorte que les bouchons (2) soient déplacés en avant le long d'un chemin de transport (P) placé dans l'espace intérieur (4), ledit tunnel d'isolation (3) séparant l'espace intérieur (4) d'un espace extérieur (5) ;
- une pluralité de chariots (20) placés dans l'espace intérieur (4) ; et
- une unité d'actionnement (21) disposée dans l'espace extérieur (5) et conçue pour déplacer en avant les chariots (20) le long d'un chemin d'avance (Q) au moyen de la génération d'un champ électromagnétique ;
chaque chariot (20) comprenant au moins un poussoir (22) conçu pour interagir avec un groupe (23) de bouchons (2) comportant un ou plusieurs bouchons (2) et de telle sorte que le déplacement en avant de chaque chariot (20) le long d'une partie fonctionnelle (Q1) du chemin d'avance (Q) corresponde au déplacement en avant du groupe (23) respectif le long du chemin de transport (P), ladite au moins une partie fonctionnelle (Q1) étant disposée dans l'espace intérieur (4) ;
l'unité d'actionnement (21) étant, pour chaque chariot (20), conçue pour commander le déplacement en avant du chariot (20) le long du chemin d'avance (Q) au moyen de la régulation du champ électromagnétique et indépendamment des autres chariots (20) ;
**caractérisée en ce que**
pour chaque chariot (20) déplacé en avant indépendamment des autres chariots (20), l'unité d'actionnement (21) est conçue pour commander par lévitation magnétique une première composante de position du chariot (20) par rapport à l'unité d'actionnement (21), ladite première composante de position étant située le long d'une direction verticale (D3), ladite direction verticale (D3) étant transversale au chemin d'avance (Q) ;
la partie fonctionnelle (Q1) étant de forme sinueuse.

2. Machine de stérilisation selon la revendication 1, dans laquelle la partie fonctionnelle (Q1) comprend une pluralité de sections linéaires (33) et une pluralité de sections courbes (34) ; dans laquelle chaque section courbe (34) relie deux sections linéaires (33) respectives l'une à l'autre.

3. Machine de stérilisation selon la revendication 2, dans laquelle chaque section courbe (34) relie une section linéaire (33) en amont respective et une section linéaire (33) en aval respective l'une à l'autre, dans laquelle la section linéaire (33) en aval est disposée en aval de la section linéaire (33) en amont respective le long de la partie fonctionnelle (Q1) ;
la machine de stérilisation (1) étant conçue de telle sorte que les chariots (22) soient déplacés en avant dans un premier sens de déplacement en avant respectif le long de la section linéaire (33) en amont respective et dans un second sens de déplacement en avant respectif le long de la section linéaire (33) en aval respective, le premier sens de déplacement en avant étant opposé au second sens de déplacement en avant.

4. Machine de stérilisation selon la revendication 2 ou 3, dans laquelle chaque section linéaire (33) est parallèle aux autres sections linéaires (33).

5. Machine de stérilisation selon l'une quelconque des revendications 2 à 4, dans laquelle au moins certaines, notamment l'ensemble, des sections linéaires (33) présentent la même longueur.

6. Machine de stérilisation selon l'une quelconque des revendications 2 à 5, dans laquelle les sections linéaires (33) sont mutuellement espacées le long d'une direction principale (Dₘ) perpendiculaire aux sections linéaires (33) elles-mêmes.

7. Machine de stérilisation selon l'une quelconque des revendications 2 à 6, dans laquelle la partie fonctionnelle (Q1) comprend quatre sections linéaires (33) ou plus.

8. Machine de stérilisation selon l'une quelconque des revendications précédentes, dans laquelle le tunnel d'isolation (3) comprend une zone d'injection (15), une zone de contact et/ou d'activation (16) disposée en aval de la zone d'injection (15) le long du chemin de transport (P) et une zone de séchage (17) disposée en aval de la zone de contact (16) le long du chemin de transport (P) ;
la machine étant conçue de façon à ce que les bouchons (2) soient exposés à un fluide de stérilisation au cours de leur déplacement en avant dans la zone d'injection (15) ;
la machine étant conçue de façon à ce que, lorsque les bouchons (2) se déplacent en avant dans la zone de contact (16), le fluide de stérilisation agisse sur les bouchons (2) ; et
la machine étant conçue de façon à ce que, lorsque les bouchons (2) se déplacent en avant dans la zone de séchage (19), le fluide de stérilisation présent sur les bouchons (2) s'évapore des bouchons (2).

9. Machine de stérilisation selon la revendication 8 et selon l'une quelconque des revendications 2 à 7, dans laquelle au moins deux sections linéaires (33) respectives de la partie fonctionnelle (Q1) se trouvent dans chacune de la zone d'injection (15), la zone de contact (16) et la zone de séchage (17).

10. Machine de stérilisation selon la revendication 8 ou 9, dans laquelle le tunnel d'isolation (3) comprend une pluralité de parois (10, 11, 12, 18, 34, 44) délimitant et/ou étant disposées dans l'espace intérieur (4) ;
la machine de stérilisation (1) comprenant une unité de stérilisation conçue pour injecter un fluide de stérilisation dans la zone d'injection (15) du tunnel d'isolation (3) ;
dans laquelle la zone d'injection (15) est délimitée par une ou plusieurs parois (10, 11, 12, 18, 34, 44) ;
dans laquelle l'unité de stérilisation (1) comprend un ou plusieurs orifices d'injection (45) et/ou une ou plusieurs buses d'injection pour injecter le fluide de stérilisation dans la zone d'injection (15) et un conduit d'alimentation pour amener le fluide de stérilisation aux orifices d'injection (45) et/ou aux buses d'injection ;
dans laquelle le conduit d'alimentation est disposé à l'intérieur d'une ou de plusieurs parois (10, 11, 12, 18, 34, 44) respectives.

11. Machine de stérilisation selon la revendication 10, dans laquelle un ou plusieurs orifices d'injection (45) et/ou une ou plusieurs buses d'injection sont raccordés à une ou plusieurs parois (10, 11, 12, 18, 34, 44) respectives ou supportés par celles-ci.

12. Machine de stérilisation selon l'une quelconque des revendications précédentes, dans laquelle la partie fonctionnelle (Q1) s'étend d'un poste de mise en prise (35), au niveau duquel chaque poussoir (22) commence, pendant l'utilisation, à interagir avec le groupe (23) respectif de bouchons (2), à un poste de libération (36), au niveau duquel chaque poussoir (22) est libéré, pendant l'utilisation, du groupe (23) respectif de bouchons (2) ;
dans laquelle l'unité d'actionnement (21) est conçue pour déplacer en avant les chariots (20) le long de la partie fonctionnelle (Q1) du chemin d'avance (Q) et le long d'une partie de retour (Q2) du chemin d'avance (Q), la partie de retour (Q2) s'étendant entre le poste de libération (36) et le poste de mise en prise (35).

13. Machine de stérilisation selon la revendication 10 ou 11, la machine de stérilisation (1) comprenant une pluralité d'éléments chauffants disposés à l'intérieur de parois (10, 11, 12, 18, 34, 44) respectives ;
dans laquelle une ou plusieurs des parois (10, 11, 12, 18, 34, 44) comprend/comprennent une garniture d'isolation thermique (46) conçue pour isoler thermiquement ou protéger l'unité d'actionnement (21) de l'élément ou des éléments chauffants.

14. Machine de stérilisation selon la revendication 13, dans laquelle chaque garniture d'isolation thermique (46) est échangeable ou remplaçable.

15. Machine de stérilisation selon l'une quelconque des revendications précédentes, dans laquelle l'unité d'actionnement (21) est définie par et/ou comprend un moteur planaire.
